# EUROPEAN PATENT SPECIFICATION

(11) **EP 4 522 301 B1**
(45) Date of publication and mention of the grant of the patent: **03.06.2026**
(21) Application number: 23727082.2
(22) Date of filing: 04.05.2023
(51) Int. Cl.: A63H 1/00, A63F 9/00, A63H 33/00, A63F 9/24

(54) **FINGER-WEARABLE ANTI-STRESS DEVICE**
AN FINGER TRAGBARE BELASTUNGSSCHUTZVORRICHTUNG
DISPOSITIF ANTI-STRESS POUVANT ÊTRE PORTÉ SUR UN DOIGT

(30) Priority: 10.05.2022 IT 202200009605
(43) Date of publication of application: 19.03.2025
(73) Proprietor: Vescovi, Christian Davide, 24043 Caravaggio, Bergamo (IT)
(72) Inventor: D'ALESSANDRO, Nicola, 20093 Cologno Monzese, Milano (IT); MOTTA, Fabrizio, 23802 Carenno, Lecco (IT)
(74) Representative: De Lorenzo, Danilo
(86) International application number: PCT/IB2023/054628
(87) International publication number: WO 2023/218292

(56) References cited:
- WO-A1-2022/060342
- CN-A- 108 187 349
- CN-U- 206 881 134
- KR-A- 20200 031 782
- US-A- 4 917 644

## Description

### Field of application

The present invention relates to a finger anti-stress device. In particular, the anti-stress device that is an object of the present invention is wearable on a finger of a user.

The finger anti-stress device according to the present invention is in the field of objects for play or stress relief.

The anti-stress device of the present invention, in particular, is suitable to be used in a wide range of applications, from the children's toys field, to the automotive gadget field, or as a keychain.

### Prior art

There are, in the art, anti-stress devices that may be used with the fingers. Some known devices are disclosed in documents CN108187349A, WO2022/060342A1, KR2020031782A. For example, some anti-stress devices in the prior art are shaped to be held by two fingers of one hand, and to be brought to move, for example rotationally, by exerting a push with the fingers of the other hand.

Disadvantageously, such known anti-stress devices are not suitable for being used with a single hand, and require both hands to be free to use them, that is, they force the user to devote both hands to their use.

Further, these devices are limited to being used for anti-stress purposes and do not provide an indication on the frequency of their use.

### Disclosure of the invention

Therefore, there appears to be a significant need to provide a finger anti-stress device capable of overcoming the drawbacks typical of the state of the art.

Thus, the present invention is intended to overcome the drawbacks of finger anti-stress devices of the prior art by providing a finger anti-stress device that may be used with a single hand, is adaptable to a wide range of functions, and in particular is suitable for providing an indication of its use.

This need is satisfied by an anti-stress device according to the independent claim 1. The dependent claims describe preferred or advantageous embodiments of the invention, comprising further advantageous features.

### Description of the drawings

The features and advantages of the finger anti-stress device will become apparent from the description below of a number of preferred embodiments, given by way of non-limiting example, with reference to the attached figures, wherein:
- Fig. 1a and 1b each show a perspective view of an anti-stress device according to one embodiment of the present invention, each from a different point of view;
- Fig. 2 shows the anti-stress device of Fig. 1, in a plan view;
- Fig. 3 shows a sectional view of the anti-stress device of Fig. 2, sectioned along the plane A-A shown in Fig. 2;
- Fig. 4 shows a detailed view of the detail B of Fig. 3;
- Fig. 5 shows a perspective view of a ring for an anti-stress device according to an embodiment of the present invention;
- Fig. 6 shows a further perspective view of the ring of Fig. 5;
- Fig. 7 shows an elevation view of the ring of Fig. 5;
- Fig. 8 shows a schematic perspective view of a printed circuit board according to an embodiment of the present invention;
- Fig. 9a and 9b each show a perspective view of a portion of the shell respectively, according to an embodiment of the present invention.

### Detailed description

With reference to the aforesaid figures, a finger anti-stress device, in particular wearable on a finger, has been indicated as a whole with the reference number 1. For clarity and ease of description, in the remainder of this discussion the finger-wearable anti-stress device will be referred to in short as an anti-stress device.

According to the present invention, the anti-stress device 1 comprises a ring 3 and a main body 10.

The ring 3 extends along and about a rotation axis Y and delimits a finger seat 300 suitable for housing at least partially a user's finger.

In the present discussion, the term "ring" refers without limitation to an object having a substantially annular body, which preferably is substantially symmetrical and hollow in the center, with either a totally open or a blind or partially blind cavity.

A ring seat 23 is obtained in the main body 10, in which the ring 3 is rotationally engaged with respect to the main body 10, to allow free rotation of the main body 10 with respect to the ring 3 and about said rotation axis Y due to a torque imposed by the user's finger on the ring 3.

Preferably, the ring seat 23 is a through cavity obtained in the main body 10.

In one embodiment, the ring 3 is engaged in the ring seat 23 by means of magnetic coupling.

The anti-stress device 1 further comprises a power supply device 90, such as a battery, and a revolution counter sensor 40, which is supplied by said power supply device 90. The revolution counter sensor 40 is integral in rotation with the main body 10 or with the ring 3 and is configured to count the number of complete relative rotations between the ring 3 and the main body 10.

It is clear that the term "counting" means, without limitation, that the revolution counter sensor 40 is configured to generate a complete rotation signal at each complete relative rotation between the ring 3 and the main body 10.

Preferably, the revolution counter sensor 40 comprises an electronic unit (such as an electronic circuit or a logic unit) configured to receive the complete rotation signal generated by the revolution counter sensor 40.

In one embodiment, the electronic unit is configured to process the complete rotation signal to generate an increment of a certain numerical value of revolutions preferably stored in a storage unit.

In a second embodiment, the electronic control unit is configured to process the complete rotation signal to transform this complete rotation signal into an activation command for a viewing system or an auxiliary notification system (e.g., auditory or visual), described later in this discussion with reference to a preferred embodiment of the invention.

That is, in this embodiment, the complete rotation signal is directly transformed into an activation command without numerically increasing a numerical value of revolutions.

The power supply device 90 is housed in the main body 10 and/or on the ring 3.

In other words, when a user inserts his/her finger into the finger seat 300 and exerts a rotary thrust force on the anti-stress device 1 with the same finger inserted into the finger seat 300, the main body 10 rotates about the ring 3.

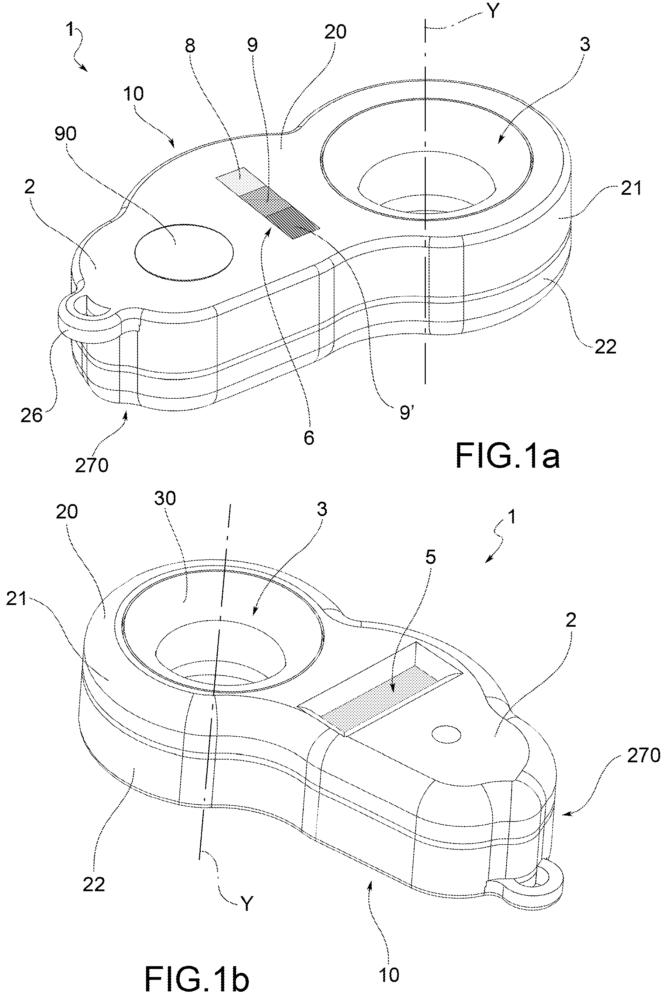

According to one embodiment, for example as shown in Fig. 1a, the power supply device 90 comprises a battery housed in the main body 10.

According to one embodiment, the power supply device comprises a system for converting mechanical power into electric power. For example, this conversion system comprises a dynamo, configured to convert a relative rotary motion between the ring 3 and the main body 10 into electric power. According to one embodiment, the conversion system is directly connected for supplying the revolution counter sensor 40. According to one variant, the conversion system is connected to a battery so that the generated electric power recharges the battery.

It is clear to the person skilled in the art that when the conversion system comprises a dynamo, it is possible for the conversion system, and thus the power device, to be housed partially on the ring 3 and partially in the main body, for example, the magnets of the dynamo on the ring 3 and the windings on the main body, or vice versa.
In other words, the conversion system makes it possible to obtain an anti-stress device 1 that is self-powering or self-charging due to the same rotary motion.

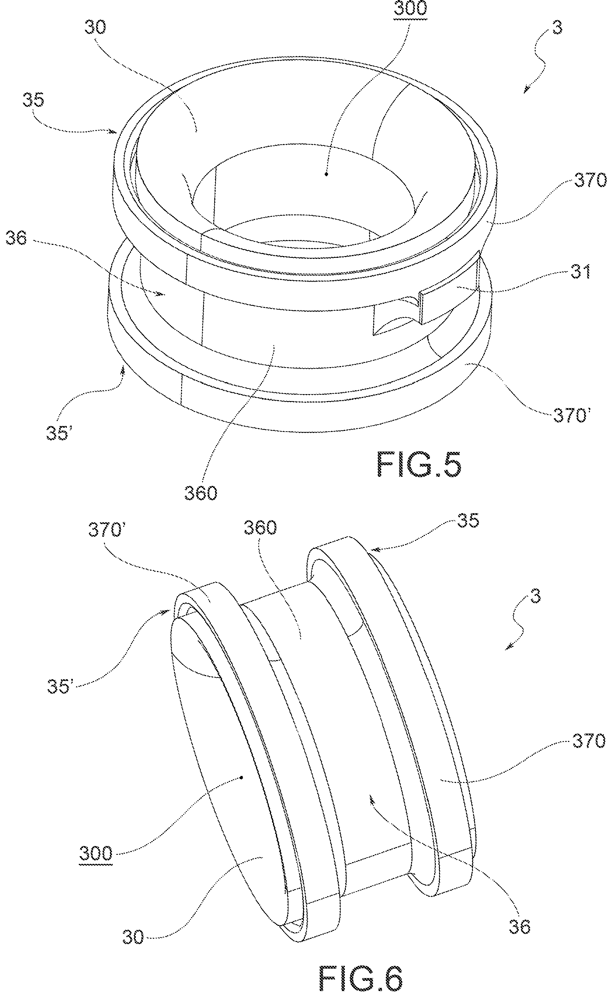

Preferably, with reference to the example embodiment shown in Fig. 5 to 7, the ring 3 is composed of a cylindrical body.

In one embodiment, the finger seat 300 is preferably a through cavity of the ring 3.

In an alternative embodiment, the finger seat 300 is a blind cavity of the ring 3, i.e., the finger seat 300 of the ring 3 is a cavity closed on one side.

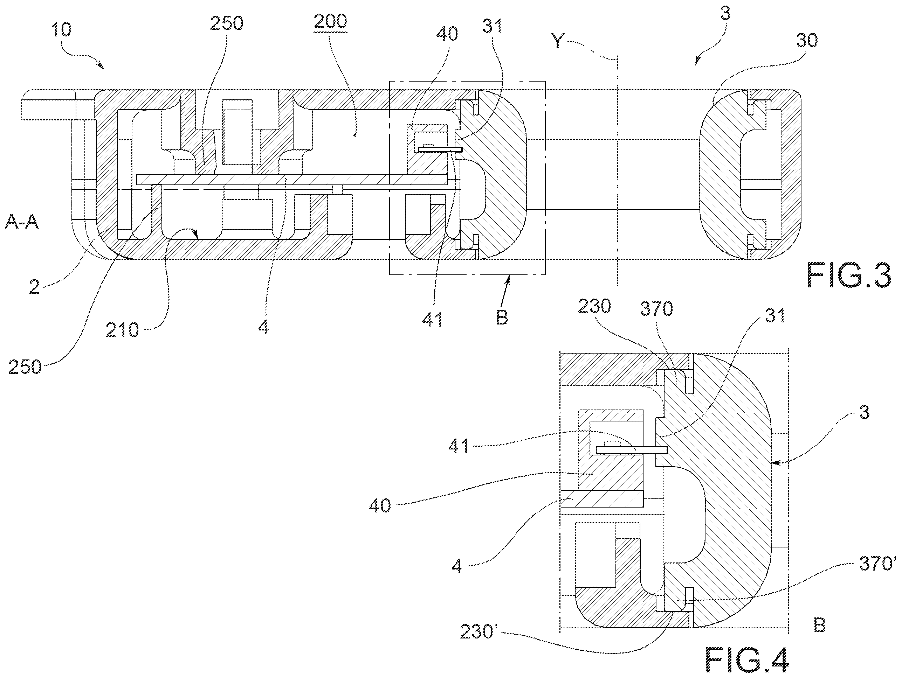

In a preferred embodiment, for example shown in Fig. 3 and 4, the revolution counter sensor 40 is contained in the main body 10 and integral therewith.

In an alternative embodiment, the revolution counter sensor 40 is placed on the ring 3 and is integral therewith.

In a preferred embodiment, the anti-stress device 1 comprises a counting lever 41 operatively connected to the revolution counter sensor 40.

According to this embodiment, the ring 3 comprises an activation tooth 31 that projects outwards therefrom, i.e., on the opposite side with respect to the finger seat 300, so that, when the user imposes a torque on the ring 3 with a finger, operating the rotation of the main body 10 about said ring 3, the activation tooth 31 is suitable for triggering the counting lever 41 at every rotation, so that the revolution counter sensor 40 is configured to increase the count of the rotation for every activation of the counting lever 41.

In a particularly advantageous embodiment, the counting lever 41 is shaped to be activated only when the rotation occurs in one direction about the rotation axis Y, and not in both directions of rotation.

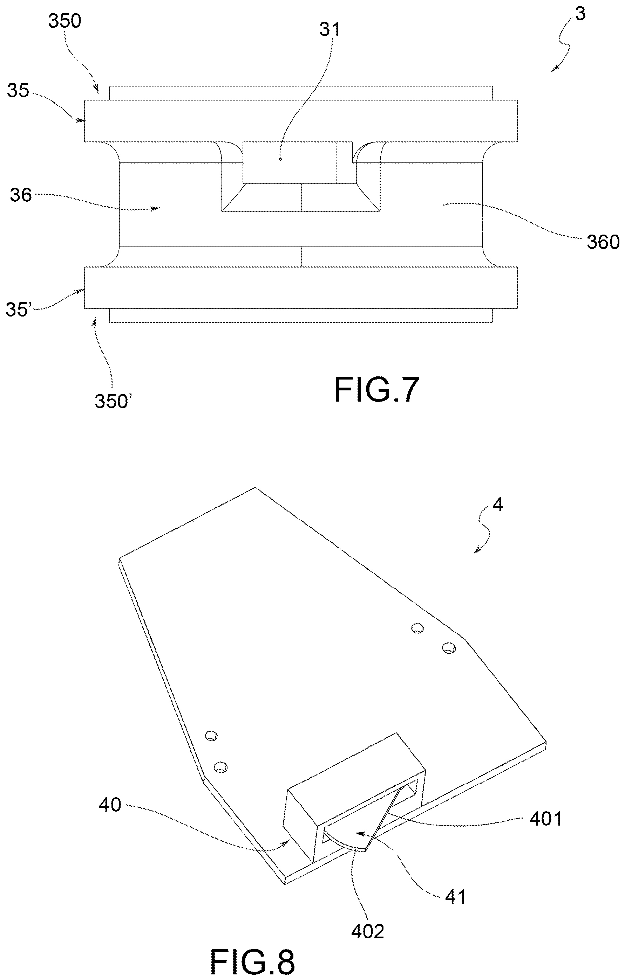

Preferably, with reference to the example in Fig. 8, the counting lever 41 is equipped with a ramp surface 401, suitable for entering in contact with the activation tooth 31 during the relative rotations between the main body 10 and the ring 3.

Further, preferably, the counting lever 41 is elastically engaged with the revolution counter sensor 40, so that the activation tooth 31, when engaged with the ramp surface 401, activates the counting lever 41 with each rotation by pressing it to switch from a protruding configuration to a pressed configuration. Further, this counting lever 41 is suitable for returning to the protruding configuration due to the elastic engagement with the revolution counter sensor 40 once the activation tooth 31 is no longer in a position to activate the counting lever 41.

In a preferred embodiment, through the elastic engagement and shape of the counting lever 41, the counting may be activated only in one direction of rotation.

In this preferred embodiment, with reference to the example in Fig. 8, the counting lever 41 preferably comprises an abutment surface 402, incident to the ramp surface 401.

The abutment surface 402 is a curved surface, or a linear surface, suitable for abutting with the activation tooth 31.

In particular, when the counting lever 41 is in a protruding configuration, if the rotation of the main body 10 about the ring 3 occurs in a first direction, the activation tooth 31 is suitable for compressing the ramp surface 401, pressing the lever and triggering the counting of the revolution counter sensor 40. If, on the other hand, the rotation of the main body 10 about the ring 3 occurs in a second direction, opposite to the first direction, the activation tooth 31 is suitable for abutting with the abutment surface 402, and not allowing the compression of the ramp surface 401.

In this embodiment, the count of revolution is only allowed in one direction of rotation, clockwise or counterclockwise, depending on how the counting lever 41 is positioned.

In an alternative embodiment, revolution counting is allowed in both directions of rotation.

According to this embodiment of the invention, the revolution counter sensor 40 comprises an electronic encoder, preferably an angle encoder, suitable for detecting the relative rotations between the ring 3 and the main body 10.

Preferably, in such an embodiment, the ring 3 does not comprise any activation teeth 31.

In an advantageous variant embodiment, for example shown in Fig. from 5 to 7, the ring 3 extends along and about the rotation axis Y between an upper end 350 and a lower end 350' and comprises an upper enlarged portion 35 at said upper end 350 and a lower enlarged portion 35' at said lower end 350'.

Further, in this variant embodiment, the ring 3 comprises a narrow central portion 36 between the upper enlarged portion 35 and the lower enlarged portion 35'.

The upper and lower enlarged portions 35, 35' extend radially with respect to the rotation axis Y by a greater thickness than the narrow central portion 36.

Preferably, as visible in the example embodiment in Fig. 7, the activation tooth 31 protrudes externally from a side wall 360 of said narrow central portion 36.

Also, preferably, the tooth 31 is made in one piece with the ring 3. That is, the tooth 31 is obtained in one piece, integrally with the ring 3.

In one embodiment of the invention, with reference to the embodiment example in Fig. 5 and 6, the upper enlarged portion 35 and the lower enlarged portion 35' each comprise a respective annular relief 370, 370'.

In this embodiment, and as shown in the example in Fig. 3, and better depicted in the enlargement in Fig. 4, the ring seat 23 is delimited by an upper seat edge 230 and a lower seat edge 230', suitable for abutting with the respective annular relief 370, 370' of the ring 3 for blocking the axial sliding of the ring 3 in the ring seat 23 along the rotation axis Y.

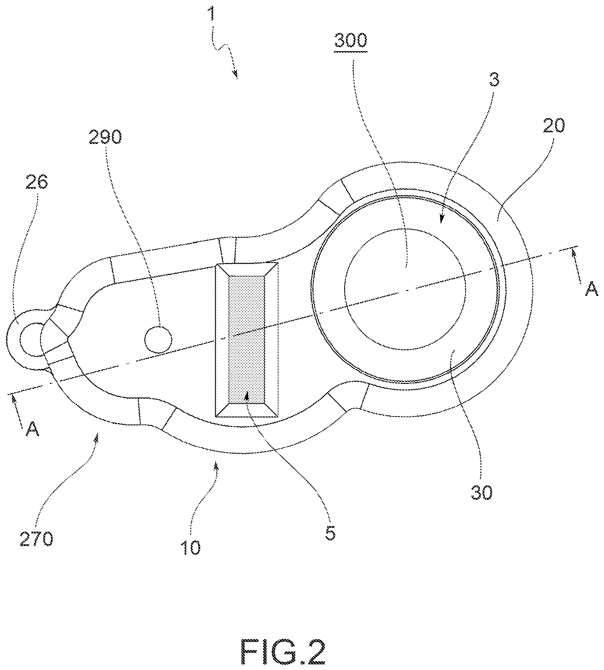

According to a preferred variant embodiment, and as visible in the example embodiment in Fig. 2, moreover, the anti-stress device comprises a viewing system 5, preferably a display, operatively connected to the revolution counter sensor 40, and suitable for showing digitally the number of rotations counted by the revolution counter sensor 40.

In a preferred embodiment, further, the main body 10 comprises a hooking means 26 suitable for hooking one or more keys.

Advantageously, this embodiment allows the anti-stress device 1 to be used simultaneously as a keychain and as a toy. Advantageously, in fact, the main body 10 is rotatable about the ring 3 even when the keys are hooked to the hooking means 26.

In a preferred embodiment, and as visible in the example embodiment in Fig. 1a the anti-stress device 1 comprises an auxiliary viewing system 6, preferably an LED strip, operatively connected to the revolution counter sensor 40. This auxiliary viewing system 6 is configured to provide a graphic representation of the stress level of the user as a function of the number of rotations counted by the revolution counter sensor 40.

In a preferred embodiment, the anti-stress device 1 comprises both the viewing system 5 and the auxiliary viewing system 6.

Preferably, the viewing system 5 and auxiliary viewing system 6 are placed on the same side of the anti-stress device 1. That is, the user sees both viewing systems from a single perspective.

Alternatively, and with reference to the example embodiment shown in Fig. 1a and 1b, the viewing system 5 and the auxiliary viewing system 6 are positioned on opposite sides of the anti-stress device 1. That is, the user sees the viewing system 5 from a first perspective, and the auxiliary viewing system 6 from a second perspective. In other words, to switch from seeing the viewing system 5 to the auxiliary viewing system 6, the user has to turn over the anti-stress device 1.

The auxiliary viewing system 6 is configured to show a first coloring 8 when the number of rotations counted by the revolution counter sensor 40 is less than a certain predetermined threshold value, and to show a second coloring 9 when the number of rotations counted by the revolution counter sensor 40 is higher than said predetermined threshold value.

Preferably, moreover, with reference to Fig. 1a, the auxiliary viewing system 6 is configured to show a first coloring 8, a second coloring 9, and a third coloring 9', depending on the number of rotations counted by the revolution counter sensor 4.

Further, preferably, the revolution counter sensor 40 is configured to be automatically reset, preferably every 24 hours.

Alternatively, or additionally, the revolution counter sensor 40 is configured to be manually reset by the user according to his will.

Preferably, when the revolution counter sensor 40 is configured to be automatically reset, for example, the auxiliary viewing system 6 is a colored bar configured to light up according to the rotation number signal detected by the revolution counter sensor 40, for example, showing the colors green, yellow, and red where each color indicates a certain stress level. For example, when the number of rotations in a day exceeds a certain upper threshold value, the colored bar lights up according to said number of rotations detected by the revolution counter sensor 40 to show the color red, indicating a high level of stress. Conversely, when the number of rotations in a day does not reach a certain lower threshold value, the colored bar lights up according to said number of rotations detected by the revolution counter sensor 40 to show the color green, indicating a low stress level.

Preferably, when the number of rotations in a day is between the lower threshold value and the upper threshold value, the bar lights up according to said number of rotations detected by the revolution counter sensor 40 to show the color yellow, indicating an intermediate stress level.

In one embodiment, the main body 10 extends about the ring 3. In other words, the entire circumference of the ring 3 is at least partially surrounded by the main body 10.

In still other words, the side wall 360 of the ring 3 always faces along the circumference at least partially toward the main body 10.

In one embodiment, the ring 3 always remains bound to the main body 10. Preferably, the ring 3 may not be separated from the main body 10 during rotation. That is, the ring 3 remains bound to the main body 10 even during rotation.

Advantageously, this variant avoids the loss of the components of the anti-stress device while preventing possible damage due to separation of the ring from the main body during rotation.

According to the invention, the main body 10 comprises a shell 2.

In one embodiment, the shell 2 contains a printed circuit board 4 operatively connected to the revolution counter sensor 40.

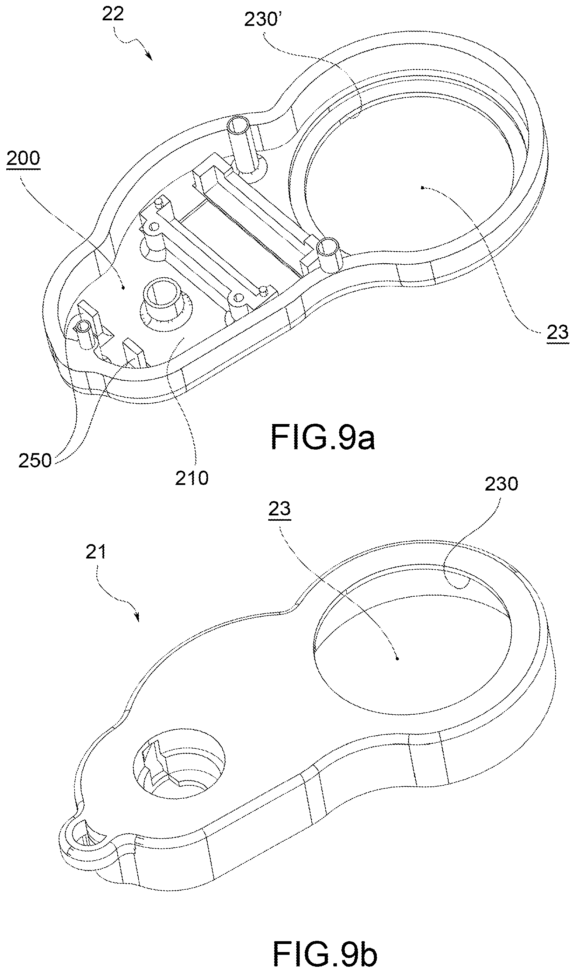

In an advantageous variant, with reference to the example embodiment shown in Fig. 1a and 1b in assembly, and in Fig. 9a and 9b in individual pieces, the shell 2 consists of a first shell portion 21 and a second shell portion 22, assembled together.

In this variant, preferably, the ring seat 23 is partially obtained in the first shell portion 21 and partially in the second shell portion 22. In other words, the ring 3 engaged in the ring seat 23 is partially engaged with the first shell portion 21 and with the second shell portion 22.

Preferably, the first shell portion 21 and the second shell portion 22 are glued together, or are attached to each other by interlocking.

In an advantageous embodiment, the shell 2 and the ring 3 are made entirely of hard plastic.

Preferably, with reference to the example in Fig. 3 and 4, the shell 2 comprises an outer wall 20 that delimits an inner cavity 200 in which the printed circuit board 4 is contained.

Further, preferably, the outer wall 20 of the shell 2 comprises an inner shell surface 210 having one or more locking protuberances 250 that constrain the printed circuit board 4 in the inner cavity 200, so that the printed circuit board 4 is locked within said inner cavity 200, even during the rotation of the main body 10 about the ring 3. In other words, these locking protuberances 250 keep the circuit board 4 in the same position, i.e., keeping it fixed, during the rotation of the main body 10 about the ring 3.

In the configuration wherein the revolution counter sensor 40 is configured to be reset, the printed circuit board 4 comprises a reset command, and on the body 2 an auxiliary opening 290 is obtained, suitable for allowing access to this reset command from the printed circuit board 4.

According to the invention, the shell 2 fully surrounds the ring 3 in the ring seat 23.

Further, with reference to Fig. 1a and 1b, in a preferred embodiment, the shell 2 comprises an elongated portion 270 that projects laterally with respect to the ring seat 23, i.e., the ring seat 23 is obtained in the main body 10 on the opposite side with respect to this elongated portion 270.

Advantageously, this preferred embodiment allows the user to effectively implement the rotation of the main body 10 with respect to the ring 3, also acting as a mass and generating a flywheel effect to facilitate rotation without requiring the application of high torque.

Preferably, the ring 3 is located at one end of the main body 10. In other words, preferably, the ring 3 is positioned away from the center of the main body 10.

That is, preferably, defining a barycenter of the anti-stress device 1, this barycenter is not located on the rotation axis Y. In other words, preferably, the ring 3 is offset from the center of gravity of the anti-stress device 1.

Advantageously, this allows the anti-stress device to be conveniently operated with one hand and to impart less torque on the ring to keep the main body rotating. This also further increased the flywheel effect that facilitates the rotation of the device.

Preferably, moreover, when the anti-stress device 1 is also configured to serve as a keychain, the hooking means 26 protrudes from the main body 10 at said elongated portion 270 of the body 2.

Further, preferably, the hooking means 26 is in one piece with the shell 2.

This configuration further increases the flywheel effect, which is greatly accentuated by the presence of keys hanging from said hooking means 26.

Preferably, there is a gap between the ring 3 and the ring seat 23.

Even more preferably, this gap is suitable to be at least partially filled with a lubricant to facilitate the relative rotation between said ring 3 and the main body 10.

Advantageously, therefore, the relative rotation between the main body 10 and the ring 3 is allowed without the need for intermediate bearings. It is clear that the person skilled in the art may still provide for intermediate bearings to allow for the relative rotation between the parts without departing from the scope of protection of the present invention.

In a preferred embodiment, the upper enlarged portion 35 and the lower enlarged portion 35' of the ring 3 comprise a curved surface 30, which is seamlessly connected to the outer wall 20 of the shell 2.

Advantageously, this shape, in addition to giving an aesthetically pleasing appearance to the product, provides greater ergonomics thereto, making it more practical to use.

Preferably, moreover, the ring has a smooth inner surface, free of roughness, so that a finger may be inserted into the finger seat 300 without causing discomfort to the user.

Preferably, the shell 2 features a design or is covered with a printed film on which a decorative motif is depicted.

In a preferred embodiment, the anti-stress device 1 further comprises light-signaling means, such as LEDs or laser emitters, installed on the ring 3 or the main body 10, suitable for emitting light during rotations of the main body 10 with respect to the ring 3.

That is, in a preferred embodiment, the anti-stress device 1 is equipped with additional lights, which generate light games, either intermittently, or continuously, or with color changes, depending on the user's rotational actuation of the main body 10 with respect to the ring 3.

In a variant embodiment, the anti-stress device 1 comprises additional sensors, installed on the ring 3 or on the main body 10, suitable for detecting vital parameters of the individual who is using the device, such as respiratory rate, heart rate, blood saturation, and blood pressure.

For example, the additional sensors comprise a heart rate monitor and/or a pressure sensor, and/or a sensor configured to detect the saturation level.

Innovatively, the finger-wearable anti-stress device according to the present invention makes it possible to resolve all the drawbacks of the anti-stress devices of the prior art.

Advantageously, the anti-stress device according to the present invention is of simple design, comprising a limited number of components.

Further, advantageously, the anti-stress device according to the present invention is usable by one hand only, and thus allows the user to have the other hand free to perform other activities.

According to a further advantage, the anti-stress device according to the present invention allows for tracking the use of the anti-stress device through the use of a revolution counter sensor.

Further, advantageously, the count operated by the revolution counter sensor is effectively shown to the user, for example, digitally, by means of a display with numbers, and/or graphically, for example, by means of an LED strip or colored bar.

Advantageously, further, the anti-stress device lends itself to a wide range of applications, such as a keychain, through the hooking means.

Advantageously, further, the anti-stress device is ergonomic, due to the shape that connects the ring and the main body in continuity, and is convenient to use.

It is clear that, to the embodiments of the aforesaid anti-stress device, a person skilled in the art, in order to meet specific needs, could make variations or substitutions of elements with functionally equivalent ones. Such variants are also included within the scope of protection as defined by the following claims. Moreover, each variant described as belonging to a possible embodiment may be implemented independently of the other variants described.

### List of reference numbers:

1 anti-stress device
10 main body
Y rotation axis
2 shell
20 outer wall
21 first shell portion
22 second shell portion
23 ring seat
26 hooking means
200 inner cavity
210 inner shell surface
230 upper seat edge
230' lower seat edge
250 locking protuberances
270 elongated portion
290 auxiliary opening
3 ring
30 curved surface
31 activation tooth
33 relief
35 upper enlarged portion
35' lower enlarged portion
36 narrow central portion
300 finger seat
350 upper end
350' lower end
360 side wall
370, 370' annular relief
4 printed circuit board
40 revolution counter sensor
41 counting lever
401 ramp surface
402 abutment surface
5 viewing system
6 auxiliary viewing system
8 first coloring
9 second coloring
9' third coloring
90 power supply device

## Claims

1. A finger-wearable anti-stress device (1), comprising a ring (3) and a main body (10),
wherein the ring (3) extends along and about a rotation axis (Y) and delimits a finger seat (300) suitable for housing at least partially a user's finger,
wherein a ring seat (23) is obtained on the main body (10), in which the ring (3) is rotationally engaged with respect to the main body (10) to allow the free rotation of the main body (10) with respect to the ring (3) and about said rotation axis (Y) due to a torque imposed by the user's finger on the ring (3),
said anti-stress device (1) comprising a power supply device (90), e.g., a battery, housed in the main body (10) and/or on the ring (3), and a revolution counter sensor (40), supplied by said power supply device (90) and rotationally integral with the main body (10) or the ring (3), said revolution counter sensor (40) being configured to count the number of complete relative rotations between the ring (3) and the main body (10),
**characterized in that** the main body (10) comprises a shell (2) which fully surrounds the ring (3) in the ring seat (23).

2. Anti-stress device (1) according to claim 1, wherein the revolution counter sensor (40) is contained in the main body (10) and is integral therewith.

3. Anti-stress device (1) according to claim 2, comprising a counting lever (41) operatively connected to the revolution counter sensor (40), and wherein the ring (3) comprises an activation tooth (31) which projects outwards and integrally therewith, on the opposite side with respect to the finger seat (300), so that, when the user imposes a torque on the ring (3) with a finger, operating the rotation of the main body (10) about said ring (3), said activation tooth (31) is suitable for triggering said counting lever (41) at every rotation, so that the revolution counter sensor (40) is configured to increase the count of the rotations for every activation of the counting lever (41).

4. Anti-stress device (1) according to any one of the preceding claims, wherein the ring (3) extends along and about the rotation axis (Y) between an upper end (350) and a lower end (350'), and comprises an upper enlarged portion (35) at said upper end (350), a lower enlarged portion (35') at said lower end (350') and comprises a narrow central portion (36) between said upper enlarged portion (35) and said lower enlarged portion (35'), wherein the upper and lower enlarged portions (35, 35') extend radially with respect to the rotation axis (Y) over a greater thickness than the narrow central portion (36).

5. Anti-stress device (1) according to claims 3 and 4, wherein the activation tooth (31) projects outwards from a side wall (360) of said narrow central portion (36).

6. Anti-stress device (1) according to claim 4 or 5, wherein the upper enlarged portion (35) and the lower enlarged portion (35') each comprise a respective annular relief (370, 370'),
wherein the ring seat (23) is delimited by an upper seat edge (230) and by a lower seat edge (230'), said upper and lower seat edges (230, 230') being suitable for abutting with the respective annular relief (370, 370') of the ring (3) for locking the axial sliding of the ring (3) in the ring seat (23) along the rotation axis (Y).

7. Anti-stress device (1) according to any one of the preceding claims, comprising a viewing system (5), for example a display, operatively connected to the revolution counter sensor (40), and suitable for showing digitally the number of rotations counted by the revolution counter sensor (40).

8. Anti-stress device (1) according to any one of the preceding claims, wherein the main body (10) comprises a hooking means (26) suitable for hooking one or more keys.

9. Anti-stress device (1) according to any one of the preceding claims, comprising an auxiliary viewing system (6), e.g., an LED strip, operatively connected to the revolution counter sensor (40), said auxiliary viewing system (6) being configured to provide a graphic representation of the stress level of the user as a function of the number of rotations counted by the revolution counter sensor (40), wherein the auxiliary viewing system (6) is configured to show a first coloring (8) when the number of rotations counted by the revolution counter sensor (40) is less than a certain predetermined threshold value, and to show a second coloring (9) when the number of rotations counted by the revolution counter sensor (40) is higher than said predetermined threshold value.

10. Anti-stress device (1) according to any one of the preceding claims, wherein the shell (2) contains a printed circuit (4) operatively connected to the revolution counter sensor (40) and wherein the shell (2) comprises an outer wall (20) delimiting an inner cavity (200) in which the printed circuit (4) is contained.

11. Anti-stress device (1) according to any one of the preceding claims, wherein the shell (2) consists of a first shell portion (21) and a second shell portion (22), assembled together, and wherein the ring seat (23) is partially obtained in the first shell portion (21) and partially in the second shell portion (22), so that the ring (3) engaged in said ring seat (23) is partially engaged with the first shell portion (21) and the second shell portion (22).

12. Anti-stress device (1) according to any one of claims 10 to 11, wherein the shell (2) comprises an elongated portion (270) projecting laterally with respect to the ring seat (23), i.e., the ring seat (23) is obtained in the main body (10) on the opposite side with respect to said elongated portion (270), and wherein the ring (3) is positioned at one end of the main body (10).

13. Anti-stress device (1) according to any one of the preceding claims, wherein between the ring (3) and the ring seat (23) there is a gap for facilitating the relative rotation between said ring (3) and the main body (10).

14. Anti-stress device (1) according to claims 4 and 10, wherein the upper enlarged portion (35) and the lower enlarged portion (35') of the ring (3) comprise a curved surface (30), which is seamlessly connected to the outer wall (20) of the shell (2).

15. Anti-stress device (1) according to any one of the preceding claims, wherein the power supply device (90) comprises a system for converting mechanical power into electric power, suitable for converting a relative rotary motion between the ring (3) and the main body (10) into electric power for supplying the revolution counter sensor (40).

## Patentansprüche

1. An Finger tragbare Belastungsschutzvorrichtung (1), umfassend einen Ring (3) und einen Hauptkörper (10),
wobei sich der Ring (3) entlang und um eine Drehachse (Y) erstreckt und einen Fingeraufnahmesitz (300) begrenzt, der geeignet ist, mindestens teilweise einen Finger eines Benutzers aufzunehmen,
wobei ein Ringsitz (23) in dem Hauptkörper (10) erhalten wird, in dem der Ring (3) drehbar in Bezug auf den Hauptkörper (10) in Eingriff steht, um die freie Drehung des Hauptkörpers (10) in Bezug auf den Ring (3) und um die Drehachse (Y) infolge eines Drehmoments zu erlauben, das durch den Finger des Benutzers auf den Ring (3) ausgeübt wird,
wobei die Belastungsschutzvorrichtung (1) eine Stromversorgungsvorrichtung (90), beispielsweise eine Batterie, aufweist, die in dem Hauptkörper (10) und/oder an dem Ring (3) aufgenommen ist, sowie einen Umdrehungszählersensor (40), der durch die Stromversorgungsvorrichtung (90) versorgt wird und drehsymmetrisch mit dem Hauptkörper (10) oder dem Ring (3) ist, wobei der Umdrehungszählersensor (40) dazu konfiguriert ist, die Anzahl vollständiger relativer Drehungen zwischen dem Ring (3) und dem Hauptkörper (10) zu zählen,
**dadurch gekennzeichnet, dass** der Hauptkörper (10) eine Schale (2) umfasst, die den Ring (3) in dem Ringsitz (23) vollständig umgibt.

2. Belastungsschutzvorrichtung (1) nach Anspruch 1, wobei der Umdrehungszählersensor (40) in dem Hauptkörper (10) enthalten ist und mit diesem einstückig ausgebildet ist.

3. Belastungsschutzvorrichtung (1) nach Anspruch 2, umfassend einen Zählhebel (41), der operativ mit dem Umdrehungszählersensor (40) verbunden ist, und wobei der Ring (3) einen Aktivierungszahn (31) umfasst, der nach außen vorsteht und einstückig mit diesem ausgebildet ist, auf der gegenüberliegenden Seite in Bezug auf den Fingeraufnahmesitz (300), so dass, wenn der Benutzer mit einem Finger ein Drehmoment auf den Ring (3) ausübt und dadurch die Drehung des Hauptkörpers (10) um den genannten Ring (3) aktiviert, der Aktivierungszahn (31) geeignet ist, den Zählhebel (41) bei jeder Drehung zu betätigen, so dass der Umdrehungszählersensor (40) dazu konfiguriert ist, die Zählung der Drehungen bei jeder Betätigung des Zählhebels (41) zu erhöhen.

4. Belastungsschutzvorrichtung (1) nach einem der vorhergehenden Ansprüche, wobei sich der Ring (3) entlang und um die Rotationsachse (Y) zwischen einem oberen Ende (350) und einem unteren Ende (350') erstreckt und einen oberen erweiterten Abschnitt (35) an dem oberen Ende (350), einen unteren erweiterten Abschnitt (35') an dem unteren Ende (350') umfasst und einen schmalen Mittelabschnitt (36) zwischen dem oberen erweiterten Abschnitt (35) und dem unteren erweiterten Abschnitt (35') umfasst, wobei sich die oberen und unteren erweiterten Abschnitte (35, 35') radial in Bezug auf die Drehachse (Y) über eine größere Dicke als der schmale Mittelabschnitt (36) erstrecken.

5. Belastungsschutzvorrichtung (1) nach den Ansprüchen 3 und 4, wobei der Aktivierungszahn (31) von einer Seitenwand (360) des schmalen Mittelabschnitts (36) nach außen vorsteht.

6. Belastungsschutzvorrichtung (1) nach Anspruch 4 oder 5, wobei der obere erweiterte Abschnitt (35) und der untere erweiterte Abschnitt (35') jeweils einen entsprechenden ringförmigen Vorsprung (370, 370') umfassen,
wobei der Ringsitz (23) durch einen oberen Sitzrand (230) und durch einen unteren Sitzrand (230') begrenzt wird, wobei die obere und die untere Sitzränder (230, 230') geeignet sind, mit den entsprechenden ringförmigen Vorsprüngen (370, 370') des Rings (3) in Anlage zu kommen, um das axiale Gleiten des Rings (3) in dem Ringsitz (23) entlang der Drehachse (Y) zu verriegeln.

7. Belastungsschutzvorrichtung (1) nach einem der vorhergehenden Ansprüche, umfassend ein Anzeigesystem (5), beispielsweise eine Anzeige, das operativ mit dem Umdrehungszählersensor (40) verbunden ist und geeignet ist, die Anzahl der Drehungen, die durch den Umdrehungszählersensor (40) gezählt werden, digital anzuzeigen.

8. Belastungsschutzvorrichtung (1) nach einem der vorhergehenden Ansprüche, wobei der Hauptkörper (10) ein Hakenmittel (26) umfasst, das geeignet ist, einen oder mehrere Schlüssel einzuhaken.

9. Belastungsschutzvorrichtung (1) nach einem der vorhergehenden Ansprüche, umfassend ein zusätzliches Anzeigesystem (6), beispielsweise einen LED-Streifen, das operativ mit dem Umdrehungszählersensor (40) verbunden ist, wobei das zusätzliche Anzeigesystem (6) dazu konfiguriert ist, eine grafische Darstellung des Belastungsniveaus des Benutzers in Abhängigkeit von der Zahl von Drehungen, die durch den Umdrehungszählersensor (40) gezählt werden, bereitzustellen, wobei das zusätzliche Anzeigesystem (6) dazu konfiguriert ist, eine erste Färbung (8) anzuzeigen, wenn die Anzahl von Drehungen, die durch den Umdrehungszählersensor (40) gezählt werden, kleiner als ein bestimmter vorbestimmter Schwellenwert ist, und eine zweite Färbung (9) anzuzeigen, wenn die Anzahl von Drehungen, die durch den Umdrehungszählersensor (40) gezählt werden, größer als der vorbestimmte Schwellenwert ist.

10. Belastungsschutzvorrichtung (1) nach einem der vorhergehenden Ansprüche, wobei die Schale (2) eine gedruckte Schaltung (4) enthält, die operativ mit dem Umdrehungszählersensor (40) verbunden ist, und wobei die Schale (2) eine Außenwand (20) umfasst, die einen inneren Hohlraum (200) begrenzt, in dem die gedruckte Schaltung (4) enthalten ist.

11. Belastungsschutzvorrichtung (1) nach einem der vorhergehenden Ansprüche, wobei die Schale (2) aus einem ersten Schalenabschnitt (21) und einem zweiten Schalenabschnitt (22) besteht, die miteinander zusammengebaut sind, und wobei der Ringsitz (23) teilweise in dem ersten Schalenabschnitt (21) und teilweise in dem zweiten Schalenabschnitt (22) erhalten wird, so dass der Ring (3), der in dem Ringsitz (23) in Eingriff steht, teilweise mit dem ersten Schalenabschnitt (21) und dem zweiten Schalenabschnitt (22) in Eingriff steht.

12. Belastungsschutzvorrichtung (1) nach einem der Ansprüche 10 bis 11, wobei die Schale (2) einen verlängerten Abschnitt (270) umfasst, der seitlich in Bezug auf den Ringsitz (23) vorsteht, das heißt, der Ringsitz (23) in dem Hauptkörper (10) auf der gegenüberliegenden Seite in Bezug auf den verlängerten Abschnitt (270) erhalten wird, und wobei der Ring (3) an einem Ende des Hauptkörpers (10) positioniert ist.

13. Belastungsschutzvorrichtung (1) nach einem der vorhergehenden Ansprüche, wobei ein Spalt zwischen dem Ring (3) und dem Ringsitz (23) vorhanden ist, um die relative Drehung zwischen dem Ring (3) und dem Hauptkörper (10) zu erleichtern.

14. Belastungsschutzvorrichtung (1) nach den Ansprüchen 4 und 10, wobei der obere erweiterte Abschnitt (35) und der untere erweiterte Abschnitt (35') des Rings (3) eine gekrümmte Oberfläche (30) umfassen, die ohne Unterbrechung mit der Außenwand (20) der Schale (2) verbunden ist.

15. Belastungsschutzvorrichtung (1) nach einem der vorhergehenden Ansprüche, wobei die Stromversorgungsvorrichtung (90) ein System zum Umwandeln mechanischer Energie in elektrische Energie umfasst, das geeignet ist, eine relative Drehbewegung zwischen dem Ring (3) und dem Hauptkörper (10) in elektrische Energie umzuwandeln, um den Umdrehungszählersensor (40) zu versorgen.

## Revendications

1. Dispositif anti-stress pouvant être porté sur un doigt (1), comprenant un anneau (3) et un corps principal (10),
dans lequel l'anneau (3) s'étend le long de et autour d'un axe de rotation (Y) et délimite un logement de doigt (300) adapté pour loger au moins partiellement un doigt d'un utilisateur,
dans lequel un logement d'anneau (23) est obtenu sur le corps principal (10), dans lequel l'anneau (3) est engagé en rotation par rapport au corps principal (10) pour permettre la rotation libre du corps principal (10) par rapport à l'anneau (3) et autour dudit axe de rotation (Y) en raison d'un couple imposé par le doigt de l'utilisateur sur l'anneau (3),
ledit dispositif anti-stress (1) comprenant un dispositif d'alimentation (90), par exemple une batterie, logé dans le corps principal (10) et/ou sur l'anneau (3), et un capteur compteur de révolutions (40), alimenté par ledit dispositif d'alimentation (90) et solidaire en rotation du corps principal (10) ou de l'anneau (3), ledit capteur compteur de révolutions (40) étant configuré pour compter le nombre de rotations relatives complètes entre l'anneau (3) et le corps principal (10),
**caractérisé en ce que** le corps principal (10) comprend une coque (2) qui entoure complètement l'anneau (3) dans le logement d'anneau (23)

2. Dispositif anti-stress (1) selon la revendication 1, dans lequel le capteur compteur de révolutions (40) est contenu dans le corps principal (10) et est solidaire de celui-ci.

3. Dispositif anti-stress (1) selon la revendication 2, comprenant un levier de comptage (41) connecté de manière opérationnelle au capteur compteur de révolutions (40), et dans lequel l'anneau (3) comprend une dent d'activation (31) qui fait saillie vers l'extérieur et est solidaire de celui-ci, du côté opposé par rapport au logement de doigt (300), de sorte que, lorsque l'utilisateur impose un couple sur l'anneau (3) avec un doigt, en actionnant la rotation du corps principal (10) autour dudit anneau (3), ladite dent d'activation (31) est adaptée pour déclencher ledit levier de comptage (41) à chaque rotation, de sorte que le capteur compteur de révolutions (40) est configuré pour augmenter le comptage des rotations pour chaque activation du levier de comptage (41)

4. Dispositif anti-stress (1) selon l'une quelconque des revendications précédentes, dans lequel l'anneau (3) s'étend le long de et autour de l'axe de rotation (Y) entre une extrémité supérieure (350) et une extrémité inférieure (350'), et comprend une portion élargie supérieure (35) au niveau de ladite extrémité supérieure (350), une portion élargie inférieure (35') au niveau de ladite extrémité inférieure (350') et comprend une portion centrale étroite (36) entre ladite portion élargie supérieure (35) et ladite portion élargie inférieure (35'), dans lequel les portions élargies supérieure et inférieure (35, 35') s'étendent radialement par rapport à l'axe de rotation (Y) avec une épaisseur plus grande que la portion centrale étroite (36).

5. Dispositif anti-stress (1) selon les revendications 3 et 4, dans lequel la dent d'activation (31) fait saillie vers l'extérieur à partir d'une paroi latérale (360) de ladite portion centrale étroite (36).

6. Dispositif anti-stress (1) selon la revendication 4 ou 5, dans lequel la portion élargie supérieure (35) et la portion élargie inférieure (35') comprennent chacune un relief annulaire respectif (370, 370'),
dans lequel le logement d'anneau (23) est délimité par un bord supérieur de logement (230) et par un bord inférieur de logement (230'), lesdits bords supérieur et inférieur de logement (230, 230') étant adaptés pour venir en butée contre les reliefs annulaires respectifs (370, 370') de l'anneau (3) pour verrouiller le glissement axial de l'anneau (3) dans le logement d'anneau (23) le long de l'axe de rotation (Y)

7. Dispositif anti-stress (1) selon l'une quelconque des revendications précédentes, comprenant un système de visualisation (5), par exemple un affichage, connecté de manière opérationnelle au capteur compteur de révolutions (40), et adapté pour montrer numériquement le nombre de rotations comptées par le capteur compteur de révolutions (40).

8. Dispositif anti-stress (1) selon l'une quelconque des revendications précédentes, dans lequel le corps principal (10) comprend un moyen d'accrochage (26) adapté pour accrocher une ou plusieurs clés.

9. Dispositif anti-stress (1) selon l'une quelconque des revendications précédentes, comprenant un système de visualisation auxiliaire (6), par exemple une bande LED, connecté de manière opérationnelle au capteur compteur de révolutions (40), ledit système de visualisation auxiliaire (6) étant configuré pour fournir une représentation graphique du niveau de stress de l'utilisateur en fonction du nombre de rotations comptées par le capteur compteur de révolutions (40), dans lequel le système de visualisation auxiliaire (6) est configuré pour montrer une première coloration (8) lorsque le nombre de rotations comptées par le capteur compteur de révolutions (40) est inférieur à une certaine valeur seuil prédéterminée, et pour montrer une seconde coloration (9) lorsque le nombre de rotations comptées par le capteur compteur de révolutions (40) est supérieur à ladite valeur seuil prédéterminée.

10. Dispositif anti-stress (1) selon l'une quelconque des revendications précédentes, dans lequel la coque (2) contient un circuit imprimé (4) connecté de manière opérationnelle au capteur compteur de révolutions (40) et dans lequel la coque (2) comprend une paroi extérieure (20) délimitant une cavité interne (200) dans laquelle le circuit imprimé (4) est contenu.

11. Dispositif anti-stress (1) selon l'une quelconque des revendications précédentes, dans lequel la coque (2) consiste en une première portion de coque (21) et une seconde portion de coque (22), assemblées ensemble, et dans lequel le logement d'anneau (23) est partiellement obtenu dans la première portion de coque (21) et partiellement dans la seconde portion de coque (22), de sorte que l'anneau (3) engagé dans ledit logement d'anneau (23) est partiellement engagé avec la première portion de coque (21) et la seconde portion de coque (22).

12. Dispositif anti-stress (1) selon l'une quelconque des revendications 10 à 11, dans lequel la coque (2) comprend une portion allongée (270) faisant saillie latéralement par rapport au logement d'anneau (23), c'est-à-dire que le logement d'anneau (23) est obtenu dans le corps principal (10) du côté opposé par rapport à ladite portion allongée (270), et dans lequel l'anneau (3) est positionné à une extrémité du corps principal (10).

13. Dispositif anti-stress (1) selon l'une quelconque des revendications précédentes, dans lequel entre l'anneau (3) et le logement d'anneau (23) il existe un jeu pour faciliter la rotation relative entre ledit anneau (3) et le corps principal (10).

14. Dispositif anti-stress (1) selon les revendications 4 et 10, dans lequel la portion élargie supérieure (35) et la portion élargie inférieure (35') de l'anneau (3) comprennent une surface courbe (30), qui est raccordée sans discontinuité à la paroi extérieure (20) de la coque (2).

15. Dispositif anti-stress (1) selon l'une quelconque des revendications précédentes, dans lequel le dispositif d'alimentation (90) comprend un système pour convertir une puissance mécanique en puissance électrique, adapté pour convertir un mouvement de rotation relative entre l'anneau (3) et le corps principal (10) en puissance électrique pour alimenter le capteur compteur de révolutions (40).
